# FASCICULE DE BREVET EUROPEEN

(11) **EP 0 657 408 B1**
(45) Date de publication et mention de la délivrance du brevet: **29.10.1997**
(21) Numéro de dépôt: 94402779.6
(22) Date de dépôt: 05.12.1994
(51) Int. Cl.: C07C 17/20, C07C 19/08

(54) **Fluoration en phase gazeuse au moyen de catalyseurs cristallisés**
Fluorierung in der Gasphase mittels kristallisierter Katalysatoren
Fluorination in the vapour phase using crystallized catalysts

(30) Priorité: 09.12.1993 FR 9314779
(43) Date de publication de la demande: 14.06.1995
(73) Titulaire: ELF ATOCHEM S.A., 92800 Puteaux (FR)
(72) Inventeur: Garcia, François, F-69530 Brignais (FR); Lacroix, Eric, F-69480 Amberieux d'Azergues (FR); Lerch, Alain, F-31400 Toulouse (FR); Rousset, Abel, F-31520 Ramonville (FR)
(74) Mandataire: Leboulenger, Jean

(56) Documents cités:
- EP-A- 0 546 883
- EP-A- 0 548 742
- EP-A- 0 554 165

## Description

La présente invention concerne le domaine de fluoration en phase gazeuse d'hydrocarbures halogénés et a plus particulièrement pour objet un procédé de fluoration catalytique d'hydrocarbures aliphatiques halogénés saturés au moyen de catalyseurs cristallisés à base de chrome.

Les recherches intenses menées sur les substituts aux chlorofluorocarbures (CFC) s'orientent, entre autres, vers la synthèse d'hydrocarbures halogénés ayant des formules plus complexes et étant chimiquement plus réactifs. Ce nouvel essor pour la fluoration a entraîné la mise au point de catalyseurs plus actifs et surtout plus sélectifs. C'est ainsi que les dérivés du chrome -déjà réputés comme de bons catalyseurs de fluoration en phase gazeuse- ont fait l'objet de préparations plus spécifiques (précurseurs, conditions opératoires...) pour améliorer encore leurs performances catalytiques. Dans certains cas, le pouvoir catalytique du chrome a également été renforcé par ajout d'autres éléments métalliques tels que cobalt, zinc, magnésium, fer, cuivre....

Dans la zone de température couramment utilisée pour la fluoration en phase gazeuse (200-450°C), la vitesse de fluoration des dérivés oxygénés du chrome par l'acide fluorhydrique décroît de manière exponentielle en fonction du taux de fluoration du catalyseur. Alors que l'on accède rapidement à un rapport atomique F/Cr de l'ordre de 1,5, l'obtention de rapports atomiques F/Cr plus élevés (2,5 à 3 dans le cas du Cr III) nécessite par contre soit des durées de fluoration très longues (> 100 heures), soit de travailler à des températures plus élevées, avec des risques de dégradation du catalyseur.

Le brevet US 3 426 009 décrit la synthèse de composés fluorés, basée sur une fluoration par HF en phase gazeuse avec un catalyseur à base de chrome. Ce catalyseur est obtenu par réduction thermique de CrO₃. L'activité du catalyseur dépend de la proportion de chrome, ayant un degré d'oxydation supérieur à 3, présent dans le catalyseur à l'issue du traitement thermique. Il est bien stipulé que cette calcination sous air ne doit pas excéder 370°C pour éviter l'obtention d'un Cr₂O₃ dénommé "oxyde de chrome vert" qui n'est pas actif pour la fluoration (colonne 1, lignes 65 à 70 et colonne 2, lignes 9 à 16).

Le catalyseur revendiqué dans le brevet US 3 992 325 est un oxyde de chrome III trihydraté. Sur ce solide longtemps considéré comme amorphe, les auteurs ont identifié une phase cristallisée orthorhombique correspondant à une structure "oxyde-hydroxyde de chrome gamma" notée "γ CrOOH". Cette phase cristallisée s'est révélée être un excellent catalyseur de fluoration (colonne 1, lignes 20 à 30). Dans la majeure partie des cas, le catalyseur doit d'abord subir une activation (colonne 3, lignes 55 à 65) et il est précisé que la température durant cette activation ne doit pas dépasser 500°C afin d'éviter la formation de toute autre phase cristallisée du chrome, inactive en fluoration (colonne 3, lignes 65 à 68 et colonne 4, lignes 1 et 2).

Une préparation spécifique d'un catalyseur au chrome décrite dans le brevet EP 514 932 A2 conduit à un oxyde de chrome de très grande surface (> 170 m²/g). Pour présenter une activité de fluoration suffisante, ce catalyseur doit être amorphe (page 3, lignes 1 à 3 et lignes 40 à 43), et donc posséder une grande surface. Les auteurs précisent qu'il est nécessaire d'éviter une calcination à trop haute température pour limiter le risque d'une cristallisation de l'oxyde de chrome (page 3, lignes 35 à 40).

A notre connaissance, seul le brevet EP 548 742 A1 revendique l'utilisation d'un catalyseur au chrome cristallisé. Ce type de catalyseur est utilisé spécifiquement pour la purification du F134a par transformation du F1122 (CF₂ = CHCl) en F133a (CF₃-CClH₂). Les catalyseurs doivent présenter un taux de cristallisation supérieur à 60 %. L'oxyde de chrome Cr₂O₃ est soit sous forme massique, soit supportée sur alumine fluorée. L'intérêt de cet oxyde de chrome cristallisé est basé sur l'absence d'émission d'oxyfluorures de chrome VI volatils et toxiques lors de la régénération (page 2, lignes 42 à 44). Pour cette purification génératrice de coke (présence de F1122), la faible surface des catalyseurs ne permet pas une durée de vie sans régénération très longue (régénération toutes les 24 heures).

L'ajout de "dopants" pour améliorer les performances catalytiques des dérivés du chrome peut également, dans certains cas, retarder la cristallisation du catalyseur. Ainsi, le brevet EP 546 883 A1 revendique un catalyseur mixte à base d'oxydes de chrome et de nickel. Bien qu'il soit possible d'introduire du Cr₂O₃ (amorphe ou cristallisé) pour améliorer la solidité du catalyseur, il est recommandé d'éviter des températures trop élevées susceptibles d'induire une cristallisation de la partie active du catalyseur (page 4, lignes 50 à 55).

De même, le brevet US 4 547 483 décrit la préparation d'un catalyseur massique par précipitation d'un sel de chrome III dopé par un sel de magnésium. De sorte à éviter une baisse de réactivité de l'oxyde de chrome vis-à-vis de l'HF, et ainsi faciliter la formation d'espèces actives fluorées, le séchage et l'activation du catalyseur sont toujours conduits à une température inférieure à 400°C (colonne 3, lignes 64 à 69).

Finalement, le brevet EP 502 605 revendique un catalyseur au chrome promu par un composé à base de zinc. Là aussi, comme pour tous les catalyseurs cités dans les exemples précédents, l'activité catalytique est liée à des espèces totalement ou partiellement fluorées. Dans ce cas précis, le catalyseur actif est constitué de fluorure de zinc, d'oxyfluorures et de fluorures de chrome (page 3, lignes 47 à 52). C'est pourquoi, il est souhaitable de prétraiter ces catalyseurs avec HF avant de les utiliser en fluoration.

Les dérivés oxygénés du chrome ayant une structure amorphe réagissent avec les agents fluorants, et plus particulièrement, avec l'acide fluorhydrique. La vitesse de fluoration de ces composés décroît exponentiellement en fonction du taux de fluoration du catalyseur. Ainsi, dans les conditions opératoires généralement utilisées en phase gazeuse (T < 450°C), la fluoration du chrome III peut se décomposer en deux étapes :
- une première fluoration rapide qui permet d'accéder en quelques heures à un rapport atomique F/Cr de l'ordre de 1,5,
- une seconde étape qui conduit de manière beaucoup plus lente (plusieurs centaines d'heures) à un rapport atomique F/Cr voisin de 3.

Cette fluoration du catalyseur par HF étant exothermique, elle nécessite une conduite très progressive (dilution dans un inerte, augmentation lente et contrôlée de la température). Autre inconvénient, elle s'accompagne d'une formation d'eau qui peut, d'une part, inhiber l'activité catalytique et, d'autre part, provoquer en présence d'hydracides une corrosion importante de l'outil industriel.

C'est pourquoi industriellement, I'étape rapide de fluoration est généralement réalisée en l'absence de produit organique susceptible de réagir avec le réactif fluorant (HF,...) ; ce traitement est couramment appelé "activation du catalyseur". Fréquemment, en raison des risques de corrosion, cette phase d'activation est conduite dans une installation simplifiée où le brut de réaction est traité directement dans des colonnes de lavage, sans récupération et recyclage du réactif fluorant qui n'a pas réagi.

Ce solide partiellement fluoré catalyse déjà les réactions de fluoration; c'est pourquoi, en raison de sa durée trop longue, la fluoration plus poussée du catalyseur au chrome a souvent lieu dans l'unité industrielle de fluoration du produit organique en présence du mélange réactionnel (organiques, HF, HCl,...). Cette perfluoration du catalyseur dans le réacteur industriel n'est pas sans inconvénients :
- elle nécessite, soit de terminer la phase d'activation dans des conditions opératoires voisines de celles de l'unité de fluoration des organiques, soit de procéder à un démarrage de l'unité industrielle très progressif, afin d'étaler dans le temps le dégagement d'eau et de calories dû à la fluoration du catalyseur. Cette exothermie vient s'ajouter à celle, inévitable, de l'adsorption d'HF sur le catalyseur,
- elle présente des risques d'emballement de la température avec dégradation de l'outil industriel et de la charge de catalyseur ; en effet, un choc thermique trop important du catalyseur fluoré se traduit souvent par une dégradation de ses propriétés mécaniques (formation de poudre),
- finalement, une formation ponctuelle de quantités importantes d'eau peut conduire à une corrosion rapide des pièces maîtresses de l'outil industriel.

A notre connaissance, jusqu'à ce jour, lors de la préparation et la mise en oeuvre de catalyseurs à base d'oxydes ou d'hydroxydes de chrome, pour obtenir des performances catalytiques acceptables, il était recommandé de conserver le caractère amorphe en évitant des traitements thermiques à des températures trop élevées (> 450°C) et sous atmosphère oxydante.

Il a maintenant été trouvé qu'à condition d'avoir une surface spécifique suffisamment importante les oxydes de chrome fortement cristallisés présentent une bonne activité pour la fluoration catalytique d'hydrocarbures aliphatiques halogénés saturés.

La présence de phases cristallisées dans le catalyseur "neuf' (vierge de tout traitement fluorant) limite sa réactivité vis-à-vis des composés fluorés, et notamment de l'HF. Après une activation rapide, les catalyseurs sont quasiment inertes vis-à-vis de l'HF. Evoluant très peu pendant leur utilisation, ils présentent l'avantage de supprimer les risques de corrosion par formation d'eau. En outre, par rapport aux catalyseurs amorphes, les catalyseurs cristallisés sont nettement moins sensibles aux chocs thermiques, notamment aux montées en température dues aux aléas de la production.

L'invention a donc pour objet un procédé de fluoration catalytique en phase gazeuse d'hydrocarbures aliphatiques halogénés saturés au moyen d'HF en présence d'un catalyseur, massique ou supporté, à base d'oxyde de chrome ou d'oxydes de chrome et d'au moins un autre métal catalytiquement actif, caractérisé en ce que la majeure partie du ou des oxydes est à l'état cristallisé et en ce que, dans le cas d'un catalyseur massique, sa surface spécifique, après activation à l'HF, est au moins égale à 8 m²/g.

Comme dans les catalyseurs amorphes de la technique antérieure, les catalyseurs cristallisés au chrome selon l'invention peuvent être dopés par la présence d'au moins un autre métal catalytiquement actif (par exemple, nickel, cobalt, manganèse, magnésium, fer, zinc, vanadium...) pour améliorer les performances catalytiques et/ou les propriétés physico-chimiques des catalyseurs. Le rapport atomique : autre métal actif/chrome est généralement inférieur à 1,2 et est de préférence compris entre 0,1 et 1.

Les catalyseurs selon l'invention peuvent être obtenus à partir des catalyseurs au chrome amorphes, en soumettant ces derniers, avant ou après leur mise en forme, à un traitement thermique dans des conditions permettant d'obtenir la cristallisation de l'espèce active et de conserver une surface spécifique suffisante (> 8 m²/g après activation par HF).

L'atmosphère de cristallisation est un paramètre important. Bien qu'on puisse travailler sous inerte (azote, argon,...), on préfère effectuer le traitement thermique en atmosphère oxydante (air, oxygène,...) qui permet d'amorcer la cristallisation à des températures plus basses et d'obtenir une surface spécifique plus importante.

La température de traitement est également importante puisqu'elle joue directement sur la cinétique de cristallisation, la taille des cristallites et sur la nature des phases cristallisées. La température minimale pour obtenir la cristallisation de l'oxyde de chrome ou des oxydes de chrome et du métal additionnel dépend des précurseurs métalliques utilisés et peut être déterminée par analyse thermique ou calorimétrique. En général, pour conserver une surface suffisante, la température du traitement sous air est comprise entre 300 et 750°C, de préférence entre 400 et 700°C ; pour un oxyde de chrome non dopé, la température est avantageusement inférieure à 650°C.

La durée du traitement thermique est bien évidemment liée aux paramètres cités précédemment, mais généralement elle n'excède pas 48 heures. Dans la majorité des cas, un chauffage de 0,5 à 24 heures permet d'accéder au solide cristallisé recherché. La montée en température est de préférence effectuée de manière progressive (20 à 200°C/heure).

Les catalyseurs amorphes de départ peuvent être préparés par les différentes techniques connues de l'homme de l'art qui permettent d'accéder à des solides de grande surface spécifique, par exemple par coprécipitation, sous forme d'hydroxydes, de différents sels métalliques ou par réduction de CrO₃. A titre non limitatif, une méthode préférée pour la préparation de catalyseurs amorphes massiques comprend les étapes suivantes :
**a)** dissolution dans l'eau d'un sel de chrome ^{III} (par exemple chlorure, sulfate ou nitrate) et éventuellement d'un précurseur d'au moins un autre métal, ce précurseur pouvant être un sel (par exemple chlorure, nitrate, acétate, sulfate,...) ou, à condition d'être soluble dans le milieu, un oxyde ou hydroxyde métallique ;
**b)** formation d'un sol par neutralisation partielle de la solution précédente au moyen d'une base (NaOH, NH₄OH, amines,...), cette neutralisation pouvant être effectuée directement dans le cas des précurseurs du type acétates ou sulfates ou nécessiter au préalable un chauffage entre 60 et 95°C dans le cas des précurseurs du type nitrates ou halogénures ;
**c)** gélification du sol par neutralisation ou basification à pH 6-11 (selon le pH de précipitation du ou des co-métaux actifs) au moyen d'une base (NaOH, NH₄OH, amines,...) ; et finalement
**d)** lavage du gel et séchage entre 50 et 200°C.

Pour parfaire la formation du sol (étape b), on peut ajouter à la solution aqueuse des précurseurs un agent complexant du chrome et/ou des métaux additionnels tel que, par exemple, I'acétate, le sulfate ou le phosphate d'ammonium, en une quantité pouvant aller jusqu'à 5 fois le nombre total de moles des précurseurs.

Le gel de l'étape c peut également être obtenu par réduction de CrO₃ en solution au moyen d'un agent réducteur tel que le méthanol.

La mise en forme du catalyseur, effectuée avant ou après l'opération de cristallisation, peut être réalisée de façon connue en soi, par exemple, par pastillage, extrusion ou granulation.

Divers additifs peuvent aussi être ajoutés durant la préparation des catalyseurs pour parfaire leurs propriétés physico-chimiques et catalytiques. On peut ainsi ajouter (% par rapport au poids du catalyseur final) :
- 2 à 30 % de poudre de Cr₂O₃ ou de Cr₂O₃, 2H₂O séchée au préalable à 300°C, ou d'Al₂O₃, xH₂O pour améliorer la tenue mécanique du catalyseur final,
- 0,1 à 5 % de graphite et/ou 0,1 à 10 % d'alcool polyvinylique pour faciliter la mise en forme par pastillage ou extrusion,
- 0,1 à 20 % de floculant tel que les polyacrylates ou polyacrylamides pour faciliter la filtration du gâteau récupéré après neutralisation.

Avec un catalyseur au chrome cristallisé selon l'invention, la phase d'activation par HF peut être largement réduite. Généralement, on la limite à une montée en température sous HF en absence d'organiques. Cependant, si le catalyseur n'a pas été cristallisé directement dans le réacteur de fluoration, il importe, avant d'introduire l'HF, de sécher le catalyseur sous inerte ou sous air, pour éliminer tout risque de corrosion. La température de ce séchage peut être comprise entre 100°C et la température utilisée pour la cristallisation ; on préfère cependant effectuer le séchage à une température comprise entre 150°C et une température inférieure de 50°C à celle à laquelle a été effectuée la cristallisation.

Ensuite, en raison de la chaleur d'adsorption de l'HF sur le catalyseur, il est recommandé d'introduire ce réactif de manière progressive, à une température comprise entre 50°C et la température fixée pour la réaction de fluoration des organiques. Généralement, on préfère effectuer l'activation à une température comprise entre 100 et 350°C, puis dès que les pics d'adsorption de l'HF sont passés, on peut monter directement à la température requise pour la réaction de fluoration du composé organique étudié. Comme le catalyseur ne risque pas de se fluorer, il n'est pas nécessaire d'observer des paliers à différentes températures. Lorsque le catalyseur est à la température souhaitée, on peut procéder à l'introduction des réactifs organiques, qui généralement viennent remplacer l'inerte utilisé pour la montée en température.

Lorsque le catalyseur contient des additifs réagissant avec les agents fluorants, il peut s'avérer nécessaire de commencer par une activation pour assurer la fluoration de ces composés. Mais étant donné les faibles proportions de ce tiers corps et généralement son aptitude à se fluorer, l'activation reste d'une durée limitée. Ce cas correspond à celui de l'alumine, qui est ajoutée pour renforcer les propriétés mécaniques du catalyseur. Elle ne s'associe pas au chrome, mais réagit avec HF pour former des oxyfluorures et du trifluorure d'aluminium. Cette alumine se fluore plus facilement que les composés à base de chrome, et une activation rapide du catalyseur par HF à la température de 350-400°C permet de fixer plus de 80 % du fluor nécessaire à sa transformation en AlF₃. Ainsi, on obtient un catalyseur qui n'évolue pratiquement plus dans le réacteur de synthèse.

Les catalyseurs cristallisés selon la présente invention peuvent être utilisés pour la fluoration par HF en phase gazeuse d'hydrocarbures aliphatiques halogénés saturés. Ils conviennent particulièrement bien à la fluoration d'hydrocarbures halogénés conduisant à des composés fluorés en C₁ à C₄ contenant un ou plusieurs atomes d'hydrogène. Comme exemples d'hydrocarbures halogénés de départ, on peut mentionner, à titre non limitatif, les composés suivants : CHCl₃, CH₂Cl₂, CH₂Cl-CFCl₂, CHCl₂-CFCl₂, CHCl₂-CClF₂, CH₂Cl-CF₂Cl, CH₃-CCl₃, CHCl₂-CF₃, CHFCl-CF₃, CH₂Cl-CF₃, CH₃-CCl₂-CH₃, CCl₃-CF₂-CH₃, CCl₃-CF₂-CHCl₂, CCl₃-CF₂-CH₂Cl, CHCl₂-CHCl-CH₃, CH₂Cl-CHCl-CH₃.

La température de la fluoration des organiques dépend de la réaction étudiée et bien évidemment des produits de réaction désirés. Ainsi, pour une substitution partielle des atomes de chlore par le fluor, on travaille à des températures comprises entre 50 et 350°C, la substitution de la totalité des atomes de chlore nécessitant généralement des températures comprises entre 300 et 500°C.

Le temps de contact dépend également de la réaction étudiée et des produits recherchés. Le plus souvent, il est compris entre 3 et 100 secondes ; cependant, pour obtenir un bon compromis entre taux de conversion et productivité, le temps de contact est avantageusement inférieur à 30 secondes.

Le rapport molaire HF/composé(s) organique(s) est également lié à la réaction étudiée. Il dépend entre autres de la stoechiométrie de la réaction. Dans la majorité des cas, il peut varier entre 1/1 et 20/1, mais là aussi, afin d'obtenir des productivités élevées, il est souvent inférieur à 10.

La pression de travail est de préférence comprise entre 1 et 20 bars absolus (0,1 à 2 MPa).

Les catalyseurs selon l'invention peuvent, suivant leur solidité mécanique, travailler en lit fixe ou en lit fluide. Les catalyseurs dont l'activité a chuté par suite d'un encrassement, peuvent être régénérés par balayage du catalyseur avec un composé susceptible d'oxyder et de transformer les produits (organiques, coke, ...) déposés sur le catalyseur en produits volatils. A ce titre, l'oxygène ou un mélange contenant de l'oxygène (air par exemple) convient parfaitement et permet de restaurer l'activité initiale du catalyseur. Il est bien sûr nécessaire de contrôler l'exothermie de cette "combustion" en contrôlant le débit d'oxygène (en début de régénération, dilution importante dans un inerte) pour prévenir un emballement de cette régénération et éviter de dépasser des températures supérieures à 600°C. Cependant, contrairement aux solides amorphes, les catalyseurs selon l'invention sont, de par leur état cristallisé, nettement moins sensibles aux élévations de température.

Pour maintenir l'activité du catalyseur, il est également possible d'effectuer la réaction de fluoration en présence d'oxygène introduit dans un rapport molaire O₂/composé organique pouvant aller de 0,001 à 0,05 et, de préférence, compris entre 0,005 et 0,03. Là aussi, les catalyseurs cristallisés sont dans un état stable qui permet de mieux supporter des températures élevées en présence d'oxygène.

Les exemples suivants illustrent l'invention sans la limiter.

### PREPARATION DES CATALYSEURS

### Catalyseur 1 (Cr₂O₃ calciné sous air à 400°C)

A 75 ml d'eau à température ambiante on a ajouté 30 g (0,075 mole) de nitrate de chrome nonahydraté Cr(NO₃)₃, 9H₂O et 32 g (0,225 mole) d'oxalate d'ammonium monohydraté, puis le mélange a été chauffé à 60°C jusqu'à dissolution totale de l'oxalate d'ammonium. La solution a ensuite été refroidie à 20°C, puis versée rapidement dans un mélange de 375 ml d'éthanol et 375 ml d'éthylèneglycol. Le précipité obtenu a été filtré, puis lavé à plusieurs reprises à l'éthanol et séché à 80°C pendant 24 heures.

Le solide obtenu a ensuite été décomposé à l'air à 400°C pendant 5 minutes, la montée en température s'effectuant à raison de 1°C/mn.

### Catalyseur 2

Même préparation que le catalyseur 1, mais en effectuant la calcination à 600°C au lieu de 400°C.

On a obtenu un catalyseur analogue au catalyseur 1 (phase cristallisée majoritaire Cr₂O₃), mais avec une surface BET de 16 m²/g.

### Catalyseur 3 (Comparatif)

On a mélangé 160 g de Cr₂O₃, 2H₂O avec 276 g d'une solution aqueuse à 12 % d'alcool polyvinylique, cet alcool facilitant le pastillage de cet oxyde de chrome cristallisé. Après séchage à 100°C pendant 15 heures, la poudre obtenue a été calcinée sous air à 400°C pendant 4 heures avant d'être mise en forme par pastillage.

### Catalyseur 4 (mixte Ni-Cr cristallisé à 600°C)

On a mélangé à température ambiante 80 ml d'une solution aqueuse 1M de nitrate de chrome nonahydraté et 80 ml d'une solution aqueuse 1M de chlorure de nickel hexahydraté. Le mélange a ensuite été chauffé à 80°C, puis refroidi avant d'y disperser 3,8 g d'Al₂O₃, 2,4H₂O. Le mélange a ensuite été neutralisé en ajoutant 50 ml d'ammoniaque 14N. Après filtration et lavage, le produit a été séché à 100°C pendant 15 heures. Au solide ainsi obtenu, on a ensuite ajouté 0,4 g de graphite et 5,1 g d'une solution aqueuse à 12 % d'alcool polyvinylique. Après un nouveau séchage à 100°C pendant 15 heures, la poudre obtenue a été calcinée sous air selon la procédure suivante :
- montée à 200°C (100°C/heure), puis palier d'une heure,
- montée à 350°C (100°C/heure), puis palier de 2 heures,
- montée à 600°C (100°C/heure), puis palier de 4 heures.

### Catalyseur 5 (mixte Ni-Cr cristallisé à 500°C)

Même préparation que le catalyseur 4 à l'exception de la température finale de calcination (500°C au lieu de 600°C).

### Catalyseur 6 (mixte Ni-Cr cristallisé à 400°C)

Même préparation que le catalyseur 4 mais avec une température finale de 400°C pour la calcination.

### Catalyseur 7 (Comparatif : mixte Ni-Cr amorphe)

Même préparation que le catalyseur 4, mais la calcination sous air jusqu'à 600°C est remplacée par un traitement thermique à 350°C sous azote.

### Catalyseur 8 (mixte Cr-V cristallisé à 500°C)

Dans 100 ml d'eau on a introduit 20 g de Cr(NO₃)₃.9H₂O et 1,57 g de VCl₃. La solution a ensuite été neutralisée jusqu'à pH 6,4 avec 14 ml d'ammoniaque 14N. Le gel ainsi formé a été lavé à l'eau, puis filtré et séché à l'étuve à 120°C pendant 14 heures. La poudre obtenue a ensuite été calcinée sous air selon la procédure suivante :
- montée à 200°C (100°C/heure), puis palier d'une heure,
- montée à 350°C (100°C/heure), puis palier de 2 heures,
- montée à 500°C (100°C/heure), puis palier de 4 heures.

### Catalyseur 9 (Comparatif : mixte Cr-V amorphe)

Même préparation que le catalyseur 8, mais en calcinant la poudre sous azote à 350°C pendant 4 heures.

### Catalyseur 10 (mixte Cr-Mg cristallisé)

200 g (0,5 mole) de nitrate de chrome nonahydraté et 26 g (0,1 mole) de nitrate de magnésium hexahydraté ont été dissous dans 1 litre d'eau. Le mélange a été chauffé à 80°C pendant 2 heures, puis basifié jusqu'à pH 10,2 par addition d'ammoniaque.

Le gel obtenu a été lavé deux fois avec 450 ml d'eau distillée, puis séché à 100°C pendant 14 heures et finalement calciné à 350°C sous air pendant 4 heures.

### Catalyseur 11 (mixte Cr-Zn cristallisé)

200 g de nitrate de chrome nonahydraté et 68 g (0,5 mole) de chlorure de zinc ont été dissous dans 1 litre d'eau. L'ensemble a été chauffé à 80°C pendant 2 heures sous agitation, puis porté à pH 6,7 par addition d'ammoniaque.

Le gel obtenu a ensuite été lavé deux fois avec 450 ml d'eau distillée, puis séché à 100°C pendant 14 heures et finalement calciné sous air à 400°C pendant 4 heures.

### Catalyseur 12 (Comparatif : mixte Cr-Zn amorphe)

Même préparation que le catalyseur 11, mais en effectuant la calcination finale à 300°C sous azote.

### ACTIVATION DES CATALYSEURS ET FLUORATION DU F133a

Les performances des catalyseurs 1 à 12, ainsi que celles d'un CrO₂ massique cristallisé (ci-après Catalyseur 13), ont été testées, après activation à l'HF, dans la fluoration du 1-chloro-2,2,2-trifluoroéthane (F133a) à pression atmosphérique.

L'acide fluorhydrique utilisé est un produit commercial ne contenant que des traces d'eau et le F133a de départ est un produit pur à 99,9 %. Le réacteur utilisé est un tube en Inconel de 20 ml chauffé par un four tubulaire.

Avant le test de fluoration, le catalyseur (15 ml) placé dans le réacteur a d'abord été séché sous azote (5 l/h) pendant 2 heures à 250°C, puis activé suivant l'un ou l'autre des modes d'activation suivants :

### (a) Activation courte à base température (pour les catalyseurs 1, 2, 3, 8 et 13)

Au courant d'azote, on ajoute progressivement de l'HF à 250°C, puis après passage des pics d'exothermicité dus à l'adsorption d'HF sur le catalyseur, ce dernier est chauffé à la température choisie pour la réaction de fluoration.

Ensuite, à cette température et sans observer de palier, on remplace progressivement l'azote par du F133a et on ajuste les débits pour obtenir le rapport molaire HF/F133a choisi.

### (b) Activation longue à basse température (pour les catalyseurs 7, 9, 10 et 12)

Même activation que précédemment, mais avant d'introduire le F133a on observe un palier de 10 heures à 350°C.

### (c) Activation à haute température (pour les catalyseurs 4, 5, 6 et 11)

Au courant d'azote, on ajoute progressivement de l'HF à 250°C, puis après passage des pics d'exothermicité, le catalyseur est chauffé à 450°C (seulement 400°C pour le catalyseur 6 et 350°C pour le catalyseur 10). Après un palier de 6 heures à cette température, on ramène celle-ci à 350°C et on remplace progressivement l'azote par du F133a pour ajuster les débits au rapport molaire HF/F133a choisi.

Avant leur introduction dans le réacteur, les réactifs sont mélangés et chauffés à la température de réaction dans un préchauffeur en Inconel.

Après lavage à l'eau (élimination des hydracides) et séchage sur CaCl₂, les produits de réaction sont analysés en ligne, par chromatographie en phase gazeuse.

Les caractéristiques de chaque catalyseur avant activation (catalyseur neuf) et après activation à l'HF sont regroupés dans le tableau I suivant. Les volumes poreux indiqués ont été déterminés par porosimétrie mercure et correspondent au volume des pores de rayon compris entre 4 nm et 63 µm.

Le tableau II rassemble les conditions opératoires et les résultats obtenus dans les tests de fluoration du F133a en F134a.

Le tableau III donne les caractéristiques des catalyseurs 4, 7 et 13 usagés, c'est-à-dire après les essais de fluoration F9, F14 et F5 respectivement.

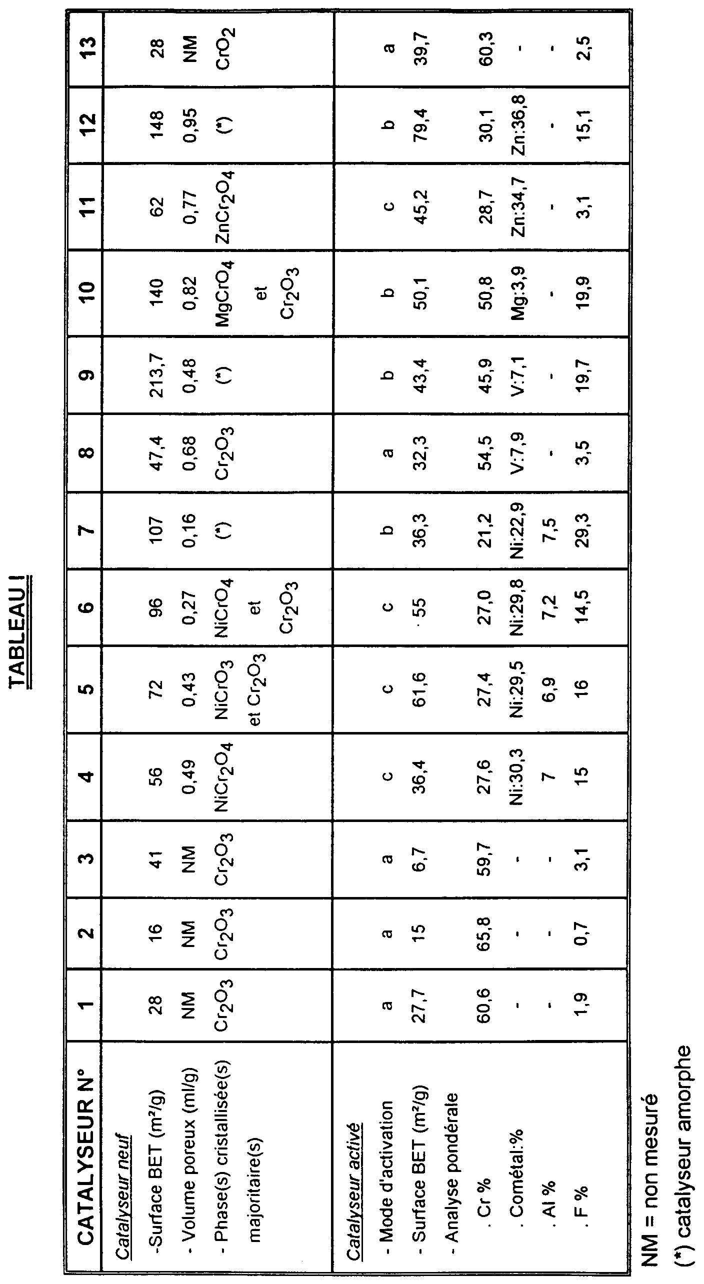

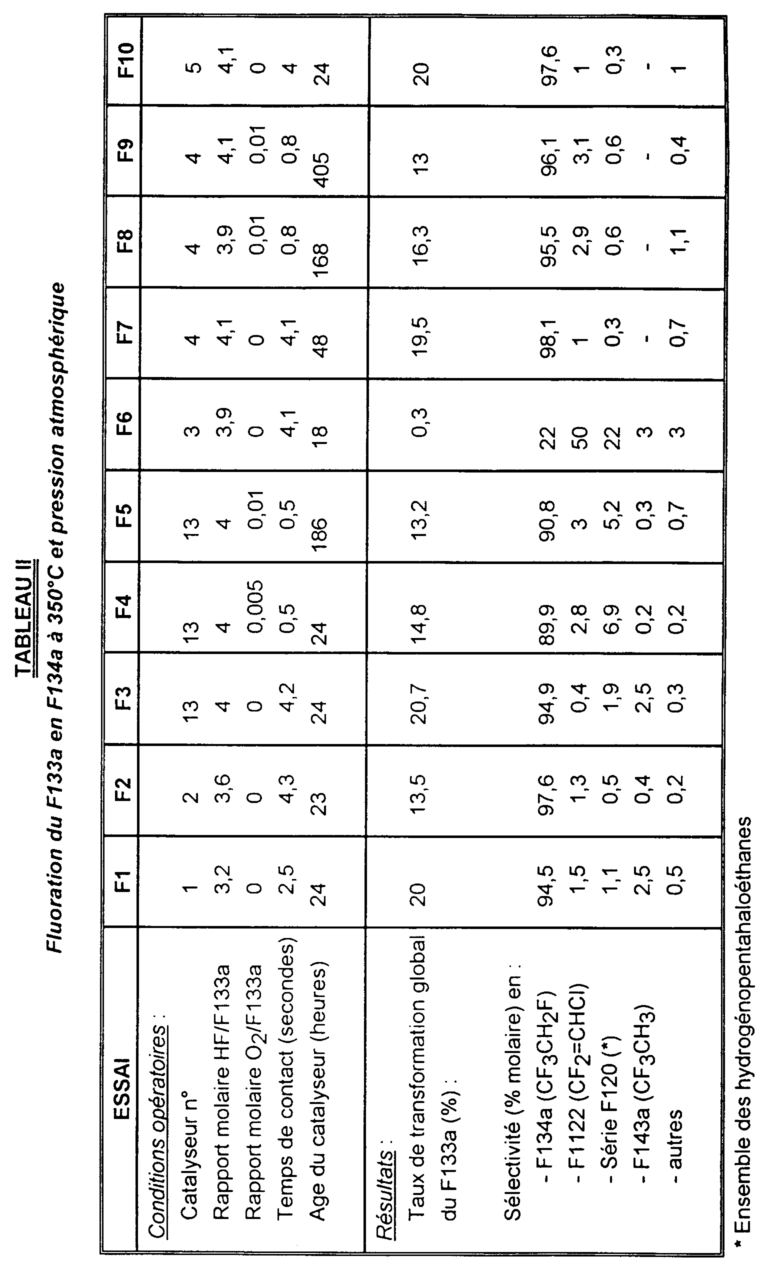

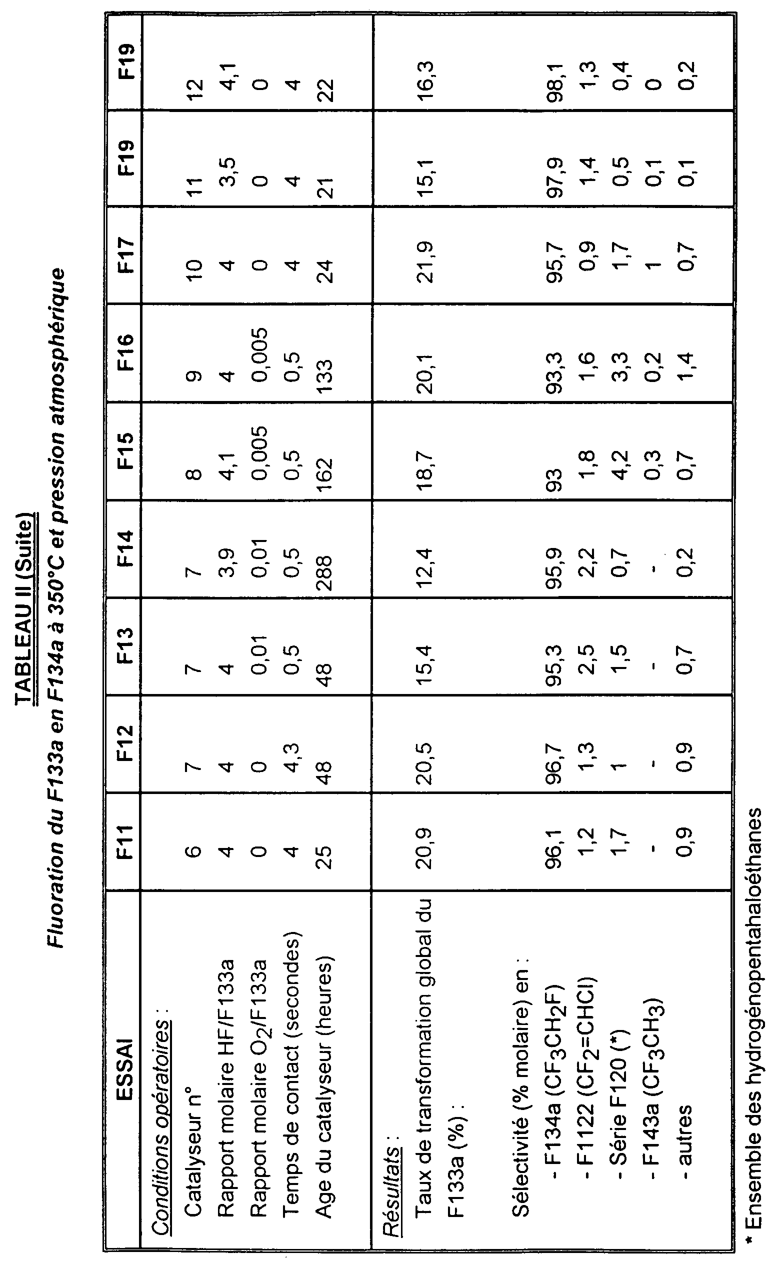

**TABLEAU III**

| ***Caractéristiques des catalyseurs usagés*** | | | |
|---|---|---|---|
| **CATALYSEUR N°** | **4** | **7** | **13** |
| Age (heures) | 405 | 288 | 186 |
| Surface BET (m²/g) | 32,4 | 25,6 | 49,4 |

| Analyse pondérale : | | | |
|---|---|---|---|
| Cr % | 25,7 | 19,2 | 60,7 |
| Ni % | 27,3 | 21 | - |
| Al % | 7 | 6,3 | - |
| F % | 18,3 | 33,5 | 3,5 |

Les essais F1, F2 et F3 montrent qu'il est possible d'obtenir de bons résultats catalytiques, proches de l'équilibre thermodynamique, avec des catalyseurs massiques cristallisés, peu fluorés mais présentant une surface après activation supérieure à 8 m²/g.

Dans les catalyseurs 1 et 2, le chrome est très majoritairement au degré d'oxydation III. Par contre, dans le catalyseur 13 des essais F3, F4 et F5, le chrome est au degré d'oxydation IV. On obtient néanmoins de très bons résultats catalytiques ; une durée de vie de 186 heures (essais F4 et F5) indique même une très bonne stabilité de cette activité.

L'essai F6 montre qu'un oxyde de chrome cristallisé présentant une surface spécifique trop faible après activation (catalyseur 3) conduit à une activité très faible.

Les essais F7 à F11 montrent l'influence de la température de cristallisation d'un catalyseur Ni-Cr massique sur son activité catalytique. Les résultats obtenus avec un temps de contact de 4 secondes (essais F7, F10 et F11) indiquent que la température optimale de cristallisation (meilleurs compromis entre fluoration du catalyseur pendant l'activation et activité catalytique) est compris entre 500 et 600°C. On constate également que le catalyseur 4 calciné à 600°C présente une activité relativement stable (essais F8 et F9) ; la légère désactivation observée est uniquement due à une amorce de cokage du catalyseur en raison d'un rapport O₂/F133a un peu faible. D'autre part, si l'on compare les taux de fluor du catalyseur 4 à différents stades (tableaux I et III), on constate que ce taux n'évolue pratiquement plus après l'activation ; le passage de 15 à 18 % en 400 heures de test correspond à la fluoration de l'alumine contenue dans le catalyseur, les constituants actifs du catalyseur (dérivés du chrome et du nickel) ne se fluorant pas ou très peu.

Testé dans des conditions opératoires similaires, le catalyseur Ni-Cr amorphe (catalyseur 7) a une activité équivalente à celle du catalyseur Ni-Cr cristallisé. Cependant, il se désactive plus rapidement et surtout sa fluoration se poursuit pendant le test de fluoration pour atteindre 33,5 % de fluor après seulement 288 heures de test.

Les essais F15 et F16 permettent de comparer les activités catalytiques de catalyseurs massiques à base de chrome et de vanadium, l'un sous forme amorphe (catalyseur 9), l'autre étant cristallisé (catalyseur 8). Bien que légèrement moins actif que son homologue amorphe, le catalyseur cristallisé présente l'avantage de se fluorer très peu : 3,5 % de fluor après activation (19,7 % pour le catalyseur amorphe).

## Revendications

1. Procédé de fluoration catalytique en phase gazeuse d'hydrocarbures aliphatiques halogénés saturés au moyen d'acide fluorhydrique en présence d'un catalyseur, massique ou supporté, à base d'oxyde de chrome ou d'oxydes de chrome et d'au moins un autre métal catalytiquement actif, caractérisé en ce que la majeure partie du ou des oxydes est à l'état cristallisé et en ce que, dans le cas d'un catalyseur massique, sa surface spécifique, après activation à l'HF, est au moins égale à 8 m²/g.

2. Procédé selon la revendication 1, dans lequel le métal associé au chrome est choisi dans le groupe constitué par les éléments suivants : magnésium, nickel, zinc, fer, cobalt, vanadium et manganèse.

3. Procédé selon la revendication 2, dans lequel le rapport atomique : autre métal actif/chrome est inférieur à 1,2 et, de préférence, compris entre 0,1 et 1.

4. Procédé selon l'une des revendications 1 à 3, dans lequel les phases cristallisées sont constituées par les oxydes Cr₂O₃, CrO₂, NiCr₂O₄, NiCrO₃, NiCrO₄, MgCrO₄, ZnCr₂O₄ ou un mélange de ces oxydes.

5. Procédé selon l'une des revendications 1 à 4, dans lequel le catalyseur est obtenu en soumettant un catalyseur amorphe à un traitement thermique dans des conditions suffisantes pour obtenir la cristallisation de l'espèce active.

6. Procédé selon la revendication 5, dans lequel le traitement est effectué sous atmosphère inerte ou oxydante à une température comprise entre 300 et 750°C, de préférence entre 400 et 700°C.

7. Procédé selon l'une des revendications 1 à 6, dans lequel, avant la fluoration de l'hydrocarbure aliphatique halogéné saturé, le catalyseur est activé au moyen d'HF à une température comprise entre 100 et 350°C.

8. Procédé selon l'une des revendications 1 à 7, dans lequel l'hydrocarbure halogéné à fluorer est un composé en C₁ à C₄ contenant un ou plusieurs atomes d'hydrogène.

9. Application du procédé selon l'une des revendications 1 à 7 à la fluoration du 1-chloro-2,2,2-trifluoroéthane en 1,1,1,2-tétrafluoroéthane.

## Claims

1. Process for the gas phase catalytic fluorination of saturated halogenated aliphatic hydrocarbons by means of hydrofluoric acid in the presence of a bulk or supported catalyst based on chromium oxide or oxides of chromium and of at least one other catalytically active metal, characterized in that the major part of the oxide(s) is in the crystalline state and in that, in the case of a bulk catalyst, its specific surface, after activation with HF, is at least equal to 8 m²/g.

2. Process according to Claim 1, in which the metal associated with the chromium is chosen from the group consisting of the following elements: magnesium, nickel, zinc, iron, cobalt, vanadium and manganese.

3. Process according to Claim 2, in which the atomic ratio: other active metal/chromium is lower than 1.2 and preferably between 0.1 and 1.

4. Process according to one of Claims 1 to 3, in which the crystalline phases consist of the oxides Cr₂O₃, CrO₂, NiCr₂O₄, NiCrO₃, NiCrO₄, MgCrO₄, ZnCr₂O₄ or a mixture of these oxides.

5. Process according to one of Claims 1 to 4, in which the catalyst is obtained by subjecting an amorphous catalyst to a heat treatment in conditions which are sufficient to obtain the crystallization of the active species.

6. Process according to Claim 5, in which the treatment is performed under an inert or oxidizing atmosphere at a temperature of between 300 and 750°C, preferably between 400 and 700°C.

7. Process according to one of Claims 1 to 6, in which, before the fluorination of the saturated halogenated aliphatic hydrocarbon, the catalyst is activated by means of HF at a temperature of between 100 and 350°C.

8. Process according to one of Claims 1 to 7, in which the halogenated hydrocarbon to be fluorinated is a C₁-C₄ compound containing one or more hydrogen atoms.

9. Application of the process according to one of Claims 1 to 7 to the fluorination of 1-chloro-2,2,2-trifluoroethane to 1,1,1,2-tetrafluoroethane.

## Patentansprüche

1. Verfahren zur katalytischen Fluorierung in der Gasphase von gesättigten, halogenierten aliphatischen Kohlenwasserstoffen mittels Fluorwasserstoff in Gegenwart eines Katalysators auf Basis von Chromoxid oder Chromoxid und einem Oxid mindestens eines weiteren katalytisch aktiven Metalls, entweder in Form eines Voll- oder Trägerkatalysators, dadurch gekennzeichnet, daß der Hauptteil des oder der Oxide in kristallisiertem Zustand vorliegt und daß im Fall eines Vollkatalysators dessen spezifische Oberfläche nach Aktivierung mit HF mindestens 8 m²/g beträgt.

2. Verfahren nach Anspruch 1, bei dem das zusammen mit dem Chrom eingesetzte Metall ausgewählt ist aus der Gruppe der folgenden Elemente: Magnesium, Nickel, Zink, Eisen, Kobalt, Vanadium und Mangan.

3. Verfahren nach Anspruch 2, bei dem das Atomverhältnis von weiterem aktiven Metall zu Chrom kleiner als 1,2 ist und vorzugsweise zwischen 0,1 und 1 liegt.

4. Verfahren nach einem der Ansprüche 1 bis 3, bei dem die kristallisierten Phasen aus den Oxiden Cr₂O₃, CrO₂, NiCr₂O₄, NiCrO₃, NiCrO₄, MgCrO₄ und ZnCr₂O₄ oder einer Mischung dieser Oxide bestehen.

5. Verfahren nach einem der Ansprüche 1 bis 4, bei dem der Katalysator erhalten wird, indem man einen amorphen Katalysator einer thermischen Behandlung unterzieht unter Bedingungen, die ausreichend sind, um eine Kristallisation der aktiven Spezies zu bewirken.

6. Verfahren nach Anspruch 5, bei dem die Behandlung unter inerter oder oxidierender Atmosphäre bei einer Temperatur zwischen 300 °C und 750 °C, vorzugsweise zwischen 400 °C und 700 °C, durchgeführt wird.

7. Verfahren nach einem der Ansprüche 1 bis 6, bei dem vor der Fluorierung des gesättigten, halogenierten aliphatischen Kohlenwasserstoffes der Katalysator mittels HF bei einer Temperatur zwischen 100 °C und 350 °C aktiviert wird.

8. Verfahren nach einem der Ansprüche 1 bis 7, bei dem der zu fluorierende, halogenierte Kohlenwasserstoff eine C₁-C₄-Verbindung ist, die ein oder mehrere Wasserstoffatome enthält.

9. Anwendung des Verfahrens nach einem der Ansprüche 1 bis 7, zur Fluorierung von 1-Chlor-2,2,2-trifluorethan zu 1,1,1,2-Tetrafluorethan.
